Europäisches Patentamt

European Patent Office

Office européen des brevets

(19)

(11) **EP 0 604 753 B1**

(12) **EUROPÄISCHE PATENTSCHRIFT**

(45) Veröffentlichungstag und Bekanntmachung des Hinweises auf die Patenterteilung:
**22.04.1998 Patentblatt 1998/17**

(51) Int. Cl.⁶: **A61M 1/14**, A61M 1/16, B01D 65/10

(21) Anmeldenummer: **93118635.7**

(22) Anmeldetag: **19.11.1993**

(54) **Verfahren und Vorrichtung zur Feststellung der Funktionsfähigkeit eines Hämo-Dialysators**

Procedure and device for ascertaining the proper functioning of a hemo-dializer

Procédé et dispositif pour vérifier le bon fonctionnement d'un hémo-dialyseur

(84) Benannte Vertragsstaaten:
**DE FR GB IT**

(30) Priorität: **27.11.1992 DE 4239937**

(43) Veröffentlichungstag der Anmeldung:
**06.07.1994 Patentblatt 1994/27**

(73) Patentinhaber:
**Fresenius Medical Care Deutschland GmbH
61350 Bad Homburg v.d.H. (DE)**

(72) Erfinder:
**Polaschegg, Hans-Dietrich, Dr.
D-61440 Oberursel (DE)**

(74) Vertreter:
**Luderschmidt, Schüler & Partner GbR
Patentanwälte,
Postfach 3929
65029 Wiesbaden (DE)**

(56) Entgegenhaltungen:
**EP-A- 0 407 737        US-A- 4 517 081**

Anmerkung:    Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Xerox (UK) Business Services
2.16.1/3.4

**Beschreibung**

Die vorliegende Erfindung bezieht sich auf ein Verfahren zur Feststellung der Funktionsfähigkeit von Baugruppen im Dialysatteil eines Dialysators (1) eines Hämodialysegerätes nach der Ansprüchen 1 und 7, und auf eine Vorrichtung, nach der Ansprüchen 6 und 9, zur Durchführung dieses Verfahrens.

Hämodialysegeräte mit volumetrischer Ultrafiltration (UF)-Kontrolle haben weite Verbreitung gefunden. Geräte dieser Bauart werden z.B. von der Anmelderin produziert und vertrieben (Hämodialysegeräte der Serie 2008 C, D, E). Die UF-Kontrolleinrichtung dieser Geräte erlaubt es, eine bestimmte Ultrafiltrationsrate bzw. Ultrafiltrationsmenge vorzugeben. Sie sorgt dann dafür, daß während der Hämodialysebehandlung die vorgegebene Ultrafiltrationsrate bzw. -menge unabhängig von der Viskosität des zu behandelnden Blutes und den Eigenschaften des Hämodialysators entzogen wird.

Es ist bekannt, daß eine solche Kontroll-Einrichtung ihr Vermögen, die Ultrafiltrationsrate exakt zu steuern, durch einen Defekt verlieren kann. Da eine durch einen solchen Defekt z.B. drastisch erhöhte Ultrafiltrationsrate zu einer Gefährdung des Patienten führen kann, verlangen bekannte Sicherheitsnormen (IEC 601 Teil 16), daß ein Schutzsystem vorhanden sein muß, das eine für den Patienten gefährliche Ultrafiltration verhindert. Als ein solches Schutzsystem wird eine Überwachung des Transmembrandruckes (TMP) akzeptiert.

Die Entwicklung von Dialyssatoren mit Membranen hoher Permeabilität, sog. high-flux Dialysatoren, hat jedoch dazu geführt, daß eine Überwachung des TMP eine gefährlich hohe oder niedrige Ultrafiltrationsrate nicht mit hinreichender Auflösung erkennen kann, bedingt durch die beschränkte Auflösung des TMP-Sensors.

Um wenigstens sicherzustellen, daß die Behandlung mit einem intakten Ultrafiltrations-Kontrollsystem beginnt, sind Einrichtungen auf dem Markt, die eine manuelle oder selbsttätige Prüfung der Integrität des Kontrollsystems vor der Behandlung erlauben. Diese Prüfung erfolgt mit einem Druckhaltetest im Dialysatteil des Gerätes.

Die Erfahrung zeigt, daß technische Geräte üblicherweise während des Betriebes und nicht im ausgeschalteten Zustand defekt werden.

Der Erfindung liegt daher die Aufgabe zugrunde, ein Verfahren zu schaffen, das mittels eines Druckhaltetestes auch eine Feststellung der Funktionsfähigkeit des Kontrollsystems für die Ultrafiltrationsrate während der Dialysebehandlung ohne zusätzliche Mittel zur Druckvariation ermöglicht.

Die Lösung dieser Aufgabe gelingt ausgehend von dem eingangs bezeichneten Verfahren dadurch, daß während der Dialyse in periodischen Zeitabständen der Dialysator für jeweils ein kurzes Zeitintervall, in welchem der mittlere Dialysatbetriebsdruck im fehlerfreien Zustand stabil ist, vom Dialysierflüssigkeitskreislauf abgetrennt wird und der Druckverlauf in der Dialysierflüssigkeit des abgetrennten Dialysators im Sinne eines an sich bekannten Druckhaltetests auf Abweichung vom stabilen Zustand erfaßt wird.

Dieses Verfahren erkennt auf einfache Weise Fehl-Ultrafiltrationen, die im aktuellen Betriebszustand gefährlich werden können.

Um realistische Verhältnisse zu haben, wird das Verfahren zweckmäßig so durchgeführt, daß das Zeitintervall,innerhalb dessen der Druckhaltetest durchgeführt wird, typischerweise im Bereich von 20 Sekunden liegt.

Da die für die Fehl-Ultrafiltration gesetzte Grenze abhängig von dem Ultrafiltrationskoeffizienten des verwendeten Dialysators bei einem Fehler zeitlich unterschiedlich erreicht wird, wird das Verfahren gemäß einer Weiterbildung der Erfindung so durchgeführt, daß die Periode zwischen zwei Druckhaltetest-Zeitintervallen abhängig von dem Ultrafiltrationskoeffizienten des eingesetzten Dialysators bestimmt wird nach der Gleichung

$$\Delta t = (\Delta UF/p * (60/UFK)$$

mit $\Delta t$ = Periode, UF = max. Fehler der Ultrafiltration,
p = Transmembrandruck und
UFK = Ultrafiltrationskoeffizient.

Das Verfahren kann manuell oder selbsttätig durchgeführt werden. Zweckmäßig ist eine Vorrichtung zur selbsttätigen Durchführung des Verfahrens erfindungsgemäß dadurch gekennzeichnet, daß im Hämodialysegerät ein Mikroprozessorsystem vorgesehen ist, der während der Dialyse periodisch in vorgegebenen Zeitabständen Schaltsignale zur Trennung des Dialysators vom Dialysierflüssigkeitskreislauf für ein vorgegebenes Zeitintervall erzeugt, dem neben dem Signal des Druckaufnehmers alle notwendigen Werte zugeführt sind und der abhängig vom Druckverlauf im Zeitintervall ein Anzeige-/Alarm-/Schaltsignal erzeugt.

Weitere ausgestaltete Merkmale der Erfindung sowie Merkmale zur Feststellung der Funktionsfähigkeit einer mit der Ultrafiltrations-Kontrolleinrichtung in Verbindung stehenden Einrichtung des Hämodialysegerätes ergeben sich aus der Beschreibung von in der Zeichnung dargestellten Ausführungsbeispielen.

Es zeigen:

Fig. 1     das Blockschaltbild eines bekannten Hämodialysegerätes, bei dem das Verfahren und die Vorrichtung nach der Erfindung bevorzugt Anwendung finden;

Fig. 2     ein Ausführungsbeispiel der erfindungsgemäßen Vorrichtung dargestellt als Blockschaltbild;

Fig. 3     den Dialysat-Druckverlauf am intakten Bilanzierungssystem des Hämodialysegerätes nach Fig. 1;

Fig. 4     das gleiche Diagramm gemäß Fig. 3 jedoch mit einem Fehler im Bilanzierungssystem;

Fig. 5     den zeitlichen Dialysat-Druckverlauf bei dem erfindungsgemäßen Druckhaltetest mit intakter Ultrafiltrations-Kontrolleinrichtung;

Fig. 6     den entsprechenden Druckverlauf wie in Fig. 5 jedoch mit einem Leck im System;

Fig. 7     ein Blockschaltbild einer Schaltung zur Ermittlung eines Fehlers im Bilanzierungssystem des Hämodialysegerätes nach Fig. 1.

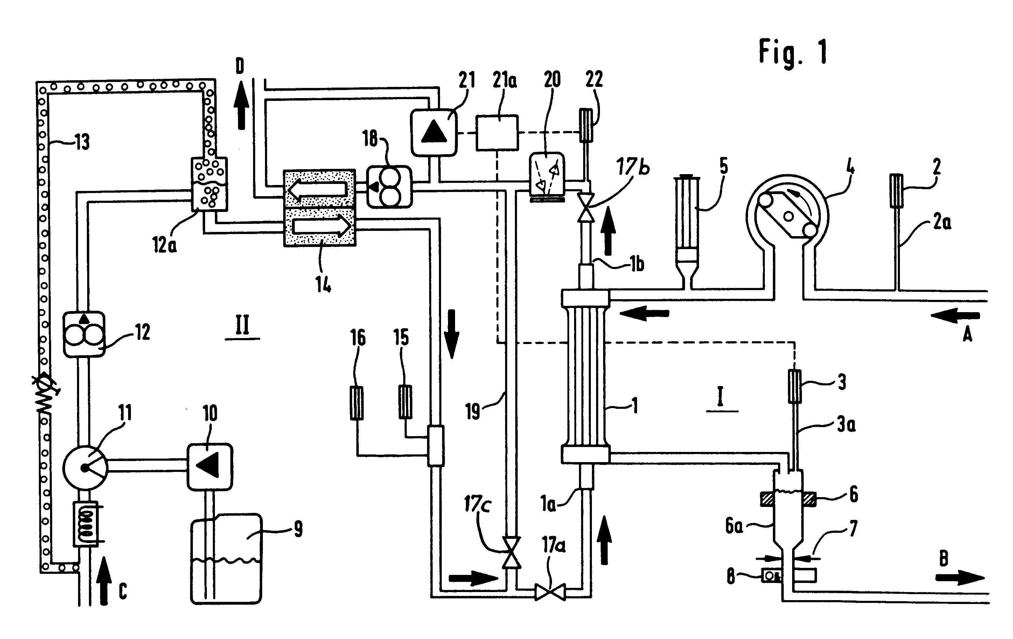

Die Fig. 1 zeigt in schematischer Darstellung (einschl. eines Flußdiagrammes) ein bekanntes Hämodialysegerät, das Gerät A 2008C der Firma Fresenius. Zentraler Bestandteil dieses Gerätes ist der Dialysator 1 mit dem extracorporalen Kreislauf I und dem Dialysierflüssigkeitskreislauf II, deren Schlauchsysteme ebenfalls nur schematisch dargestellt sind.

Dialysatoren sind in zahlreichen Varianten handelsüblich. Vorzugsweise wird ein Hemoflow-Kapillardialysator verwendet.

In dem vom Patienten zum Dialysator 1 führenden Kreislaufteil, dem Blutschlauchsystem (Richtung Pfeil A), findet zunächst eine arterielle Druckmessung statt. Die arterielle Druckmessung erfolgt mit einem piezoresistiven Druckaufnehmer 2, an den die Druckmeßleitung 2a des Blutschlauchsystems unter Zwischenschalten eines hydrophoben Membranfilters (nicht gesondert dargestellt) als Kontaminationsschutz angeschlossen wird.

In gleicher Weise erfolgt in dem zum Patienten führenden Kreislaufteil (Richtung Pfeil B) eine Messung des venösen Rücklaufdruckes mit einem Druckaufnehmer 3 über die Druckmeßleitung 3a.

Für den Blutfluß im extracorporalen Kreislauf I sorgt eine Blutpumpe 4, insbesondere in Form der notorisch bekannten peristaltischen Schlauchpumpen.

Eine Heparinpumpe 5 sorgt für die kontinuierliche Zuführung der benötigten Heparindosis, um ein Gerinnen des Blutes zu vermeiden.

Stromabwärts vom Dialysator 1 ist ferner ein Luftdetektor 6 vorgesehen, der verhindert, daß Blutschaum oder Luft, welche in den extracorporalen Kreislauf I gelangt sind, dem Patienten infundiert werden. Er enthält eine nicht dargestellte Tropfkammerhalterung, in der sich die venöse Tropfkammer 6a des Blutschlauchsystems befindet. Mit Hilfe von Ultraschallimpulsen erkennt der Luftdetektor 6, ob sich Luftblasen oder Mikroschaum in der Tropfkammer 6a befinden.

Eine elektromagnetische Schlauchabsperrklemme 7 schließt den venösen Rücklauf zum Patienten bei Gefahr unter Anhalten der Blutpumpe 4.

Ein zusätzliches optisches System 8 in der Schlauchhalterung unter der Tropfkammer 6 erkennt, ob sich Blut im Schlauch befindet oder ob Kochsalzlösung (oder evtl. auch Luft) enthalten ist.

Im Dialysierflüssigkeitskreislauf II wird aus Wasser (Pfeil C) und Konzentrat im Verhältnis 34 : 1 die Dialysierflüssigkeit für die Dialyse hergestellt, entgast und auf Körpertemperatur erwärmt.

Zu diesem Zweck wird Konzentrat aus einem Konzentratbehälter 9 mittels einer Pumpe 10 entnommen und dem Mischpunkt 11 zugeführt, wo die Mischung mit dem Wasser erfolgt. Die Entgasung erfolgt mittels einer Zahnradpumpe 12, die einen Unterdruck erzeugt. Das in der Luftabscheide-Kammer 12a ausperlende Gas wird über die Leitung 13 rückgeführt.

Die Dialysierflüssigkeit durchströmt dann den unteren Teil einer Bilanzkammer 14, in der die zum Dialysator 1 strömende, frische Dialysierflüssigkeit durch genau die gleiche Menge verbrauchter Flüssigkeit vom Dialysator ersetzt wird. Die Bilanzkammer dient außer zur Bilanzierung noch als Teil des Mischsystems. Nach jeder Füllung einer Bilanzkammer pumpt eine Membranpumpe die richtige Menge Konzentrat in den Mischpunkt. In die Zuleitung zum Dialysator 1 sind ein Temperaturfühler 15 und ein Leitfähigkeitssensor 16 zur Erfassung des Zustandes der Dialysierflüssigkeit vorgesehen. In der Zuleitung befindet sich weiterhin ein Ventil, das erste Dialysatorventil 17a. Im Ablauf des Dialysators 1 befindet sich ein weiteres Ventil, das zweite Dialysatorventil 17b. Vor dem ersten Dialysatorventil 17a zweigt eine Leitung, die Bypassleitung 19 ab, die stromab des zweiten Dialysatorventils 17b in den Kreislauf mündet. In diese Leitung 19 ist das Bypassventil 17c geschaltet. Das Ventil 17c einerseits und die Ventile 17a und 17b andererseits werden abwechselnd geschaltet. Sind die Ventile 17a und 17b geschlossen und das Ventil 17c geöffnet, ist der Dialysator 1 abgeschaltet und durch die Bypassleitung 19 überbrückt. Sind die Ventile 17a und 17b geöffnet, sowie das Ventil 17c geschlossen, dann durchströmt die Dialysierflüssgigkeit den Dialysator.

Für den Fluß der Dialysierflüssigkeit im Kreislauf II dient eine Pumpe 18, die Dialysierflüssigkeitspumpe, die in der abführenden Leitung des Dialysierflüssigkeitsteiles II angeordnet ist. Die verbrauchte Flüssigkeit durchströmt dann den oberen Teil der Bilanzkammer 14 und gelangt danach in den Abfluß (Pfeil D).

Eine Trübung der abfließenden Dialysierflüssigkeit wird von der Blutlecküberwachung 20 mit Hilfe eines Infrarot-Transmissionsverfahrens erkannt und als Blutalarm angezeigt. Damit werden Membranrupturen oder kleine Blutlecks im Dialysator festgestellt.

Eine an die Abflußleitung angeschlossene Pumpe 21, vorzugsweise eine volumetrische Membranpumpe, entzieht mit einer vorgegebenen Rate dem System Flüssigkeit. Als Folge stellt sich ein vom Dialysator abhängiger Unterdruck, der Transmembrandruck, zwischen 0 und -22 kPa (0 und -540 mmHg) ein; die durch die Membranpumpe entzogene Flüssigkeit strömt als Ultrafiltrat (UF) aus dem Blut nach und gelangt ebenfalls in den Abfluß (Pfeil D) oder in ein separates Auffanggefäß.

Der Dialysierflüssigkeitsunterdruck wird mit einem elektronischen Druckaufnehmer 22 gemessen und, subtrahiert vom venösen Rücklaufdruck am Sensor 3, als Transmembrandruck (UF), erfaßt.

Der mittlere Transmembrandruck (TMP) ist definiert als:

$$TMP = \frac{(P_{bi}+P_{bo})}{2} - \frac{(P_{di}+P_{do})}{2}$$

mit

$P_{bi}$ = Blutdruck auf der Eingangsseite des Dialysators
$P_{bo}$ = Blutdruck auf der Ausgangsseite des Dialysators
$P_{di}$ = Dialysierflüssigkeits-Druck auf der Eingangsseite 1a des Dialysators
$P_{do}$ = Dialysierflüssigkeits-Druck auf der Ausgangsseite 1b des Dialysators

Alle Komponenten des extracorporalen Blutkreislaufes I sind mit Sicherheitseinrichtungen untereinander verknüpft. Ein Absinken des Blutpegels in der Tropfkammer 6 veranlaßt den Luftdetektor 6a, die Blutpumpe 4 abzuschalten und die Schlauchabsperrklemme 7 zu schließen. Das gleiche gilt für alle auftretenden Blutalarme (Blutleckalarm, ausgelöst durch den Sensor 20, Über- oder Unterschreiten der arteriellen, venösen oder UF-Druck-Alarmgrenzen, ausgelöst durch die Sensoren 2, 3 und 20). Ferner sind (nicht dargestellte Anzeigen) für die einzelnen Meßsignale vorhanden.

Die Bilanzkammer 14 gewährleistet, daß zum bzw. vom Dialysator 1 stets die gleiche Menge Dialysierflüssigkeit zu- bzw. abgeführt wird.

Bei der konkreten Ausgestaltung der Bilanzkammer sind beide Kammerteile durch eine Membran getrennt. Die Kammerteile haben dabei das gleiche Volumen.

Die Bilanzkammer arbeitet taktweise. Im Takt 1 füllt sich die untere Seite der Bilanzkammer mit frischer Dialysierflüssigkeit. Der Druck wirkt auf die Membran, verbrauchte Dialysierflüssigkeit fließt in den Abfluß.

Bei max. Ausdehnung der Membrane entspricht das ausgestoßene Flüssigkeitsvolumen VA dem Kammervolumen.

Im Takt 2 füllt sich die obere Seite der Bilanzkammer mit verbrauchter Dialysierflüssigkeit. Die frische Dialysierflüssigkeit wird zum Dialysator gedrückt. Bei max. Ausdehnung der Membrane entspricht das ausgestoßene Flüssigkeitsvolumen VF dem Kammervolumen.

In den beiden bezeichneten Fällen ist das aus dem einen oder anderen Kammerteil ausgestoßene Flüssigkeitsvolumen genau gleich dem Gesamtvolumen der Kammer:

$$VF = Kammervolumen = VA$$

$$VF = VA$$

Die Verwendung von lediglich einer einzigen Bilanzkammer 14 würde zu einem diskontinuierlichen Dialysierflüssigkeitsfluß führen. Um einen kontinuierlichen Dialysierflüssigkeitsfluß zu erzielen, wird eine weitere (nicht dargestellte) Bilanzkammer parallel zur ersten Kammer geschaltet und in umgekehrter Sequenz betrieben.

Weitere Einzelheiten zu diesem Bilanzierungssystem können der DE-A-28 38 414 entnommen werden.

Aufgrund der erläuterten Eigenschaft der Bilanziervorrichtung 14 kann weder in der einen noch in der anderen Richtung eine Volumenverschiebung zwischen der Blut- und der Dialysatseite im Dialysator 1 stattfinden.

Die sog. Ultrafiltrationsrate wäre demzufolge Null.

Der zwischen der Bilanziervorrichtung 14 und dem Dialysator 1 eingeschlossene Teil des Flüssigkeitskreislaufes verhält sich wie ein geschlossenes volumenkonstantes System. Um aus diesem System Flüssigkeit abzuführen, ist die Pumpe 21 vorgesehen. Die mit Hilfe dieser Pumpe aus dem System abgeleitete Flüssigkeitsmenge muß aufgrund der erwähnten Eigenschaften der Bilanziervorrichtung durch eine gleichgroße Flüssigkeitsmenge ersetzt werden, die von der Blutseite I zur Dialysatseite II des Dialysators 1 übergeht. Die mittels der Pumpe 21 abgeleitete Flüssigkeitsmenge stimmt also mit der durch die Membran des Dialysators 1 tretenden Flüssigkeitsmenge, dem Ultrafiltrat, überein.

Der Pumpe 21 ist eine Kontrolleinrichtung 21a zugeordnet, mittels der eine kontrollierte volumengesteuerte Ultrafiltration erzielt werden kann. Diese Ultrafiltrations(UF)-Kontrolleinrichtung 21a erlaubt es, eine bestimmte Ultrafiltrationsrate bzw. -menge vorzugeben.

Die Volumensteuerung ermöglicht daher bei allen Dialysatortypen eine exakte Ultrafiltration. Damit ist die während der Dialyse entzogene Flüssigkeitsmenge nicht mehr vom Blutdruck, den Eigenschaften des Dialysators und dem manuell eingestellten Unterdruck abhängig.

Diese Kontrolleinrichtung 21a kann jedoch ihr Vermögen, die UF-Rate exakt zu steuern, durch einen Defekt verlieren. Sowohl eine stark erhöhte wie auch eine stark erniedrigte UF-Rate können jedoch zu einer Gefährdung des Patienten führen. Die Kontrolleinrichtung 21a weist daher ein Schutzsystem auf, das eine für den Patienten gefährliche UF-Rate verhindert. Dieses Schutzsystem basiert auf der Überwachung des Transmembrandruckes TMP, was in Fig. 1 durch die Wirkungslinien vom Kontroll-/Schutzsystem 21a zu den Sensoren 22 und 3 schematisch dargestellt ist. Dieser Transmembrandruck ändert sich typischerweise bei einem Fehler, z.B. einem Leck im UF-Kontrollsystem.

Die Entwicklung von Dialysatoren mit Membranen hoher Permeabilität, sog. high-flux Dialysatoren, hat jedoch dazu geführt, daß eine TMP-Überwachung eine gefährlich hohe UF-Rate nicht mit hinreichender Auflösung erkennen kann. Dies wird durch die folgende Abschätzung demonstriert:

Ein high-flux Dialysator hat einen Ultrafiltrationskoeffizienten (UFK) von 150-450L/h · kPa (20 - 60 l/h und mmHg). Ein üblicher TMP-Sensor hat eine Auflösung von 20 mmHg. Eine Abweichung vom Sollwert des TMP von 20 mmHg führt daher gerade noch nicht zum Alarm und bedeutet jedoch bei einem UFK von 150L/h · kPa 20L/h · mmHg) eine Mehrultrafiltration von 1 l/h, bei einem UFK von 450L/h · kPa (60 L/h · mmHg) eine solche von 3 l/h. Abweichungen von mehr als 0,5 l können aber schon unangnehme bzw. gefährliche Blutdruckabfälle beim Patienten auslösen. Ebenso gefährlich ist auch eine umgekehrte Ultrafiltration in dieser Größenordnung, da sie zu einer Überwässerung des Patienten führt.

Um nun wenigstens sicherzustellen, daß die Behandlung mit einem intakten Ultrafiltrationskontrollsystem beginnt, sind Einrichtungen bekannt, die eine manuelle oder automatische Prüfung der Integrität des Ultrafiltrations-Kontrollsystems erlauben. Dies erfolgt mit einem sog. Druckhaltetest:

Bei dem beschriebenen UF-Kontrollsystem handelt es sich um ein geschlossenes System. In diesem System wird nun mit einer zusätzlichen Pumpe ein Unter- bzw. Überdruck erzeugt und nach Abschalten der Pumpe der Druckverlauf über die Signale des Druckaufnehmers 22 beobachtet. Ändert sich der Druck nicht bzw. nur geringfügig, so ist das System dicht und somit intakt. Läßt sich kein Druck aufbauen oder ändert sich der Druck rasch, so ist dies ein Zeichen für ein Leck im System. Es ist somit defekt und das Hämodialysegerät darf nicht zur Behandlung eingesetzt werden.

Nun zeigt die Erfahrung, daß technische Geräte üblicherweise während des Betriebes und nicht im ausgeschalteten Zustand defekt werden. Der beschriebene, vor der Behandlung durchgeführte Druckhaltetest verhindert zwar, daß mit einem defekten Gerät wiederholt Patienten behandelt werden, nicht aber eine gefährliche Ultrafiltration wegen eines Defektes während der Behandlung.

Um nun einen Defekt während der Behandlung zu erkennen, ehe er gefährliche Auswirkungen zeigt, kann ein Druckhaltetest auch während der Dialyse periodisch durch eine kurzzeitige Unterbrechung der Dialyse durchgeführt werden. Dazu wird der Dialysator 1 durch die stromauf und stromab des Dialysators im Dialysierflüssigkeitskreislauf angebrachten Dialysatorventile (17a,b) isoliert. Gleichzeitig wird das Bypassventil 17c geöffnet. Danach wird ein Druckhaltetest, wie oben beschrieben, durchgeführt. Nach erfolgreich verlaufenem Test wird das Bypassventil geschlossen und die Dialysatorventile geöffnet und die Behandlung fortgesetzt.

Um generell einen negativen Druck im Dialysierflüssigkeitskreislauf zu erzeugen, bedient man sich üblicherweise der ohnehin vorhandenen Ultrafiltrationspumpe 21. Um einen positiven Druck zu erzeugen, bedarf es einer zusätzlichen Pumpe. Als solche könnte z.B. eine am Sekundärluftabscheider 12a angebrachte Luftpumpe dienen, indem sie umgekehrt betrieben wird, d.h. Luft in den Luftanscheider hineinpumpt.

Die Prüfung mit positiven und negativen Drücken ist bei v.g. Druckhaltetest initial notwendig, da zu diesem Zeitpunkt nicht bekannt ist, bei welchem Wert sich der Dialysierflüssigkeitsdruck einstellen wird. Es gibt nämlich Lecks, die nur in einem Druckbereich wirksam werden.

Der beschriebene Druckhaltetest bedürfte somit gegenüber dem Gerät nach Fig. 1 einer zusätzlichen Pumpe, wäre zeitaufwendig und führte zu einem Ultrafiltrationsfehler. Außerdem deckte er u.U. ein Leck auf, das akut gar nicht gefährlich ist, da es beim aktuellen Betriebsdruck nicht wirksam ist. Die Dialyse-Behandlung würde dann unnötigerweise abgebrochen.

Die Erfindung sieht ein Verfahren vor, das auf zusätzliche Hilfsmittel zur Erzeugung von positiven bzw. negativen Druckabweichungen verzichtet. Dazu wird der Druckhaltetest periodisch während der Dialyse beim Betriebsdruck durchgeführt. Dazu wird, wie bereits beschrieben, der Dialysator 1 vom Dialysierflüssigkeits-Kreislauf getrennt, das Bypassventil 17 geöffnet und der Betriebsdruck-Verlauf anhand des Verlaufes des Signales am Druckaufnehmer 22 beobachtet. Dieses Verfahren ist nicht nur einfacher, sondern es erkennt auch nur Fehlultrafiltrationen, die im aktuellen Betriebszustand gefährlich werden können. Durch eine relativ kurzfristige Beobachtung des Druckverlaufes können Lecks mit ca. 0,125 l/h ohne weiteres erkannt werden.

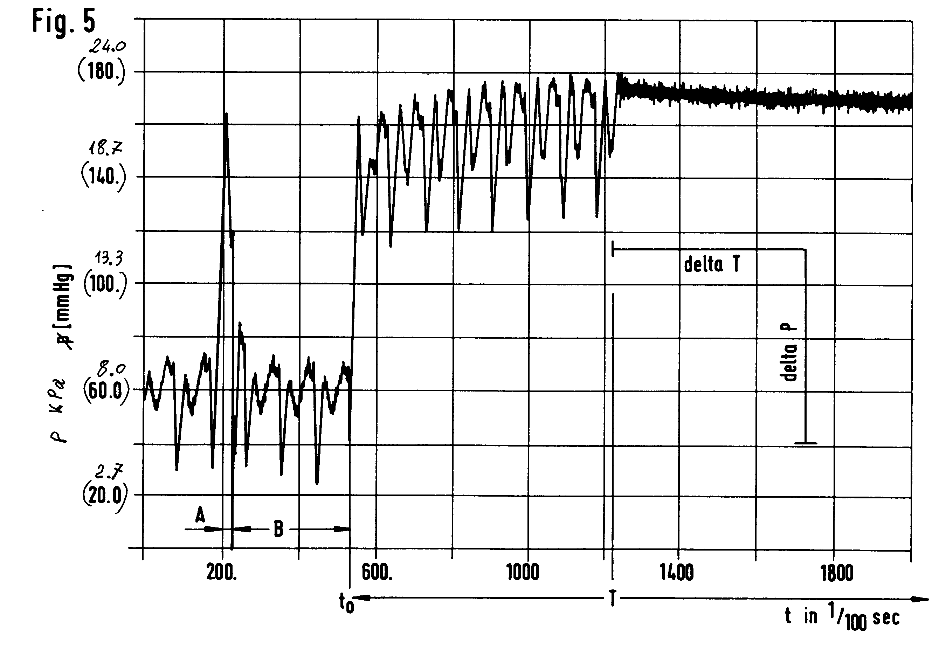

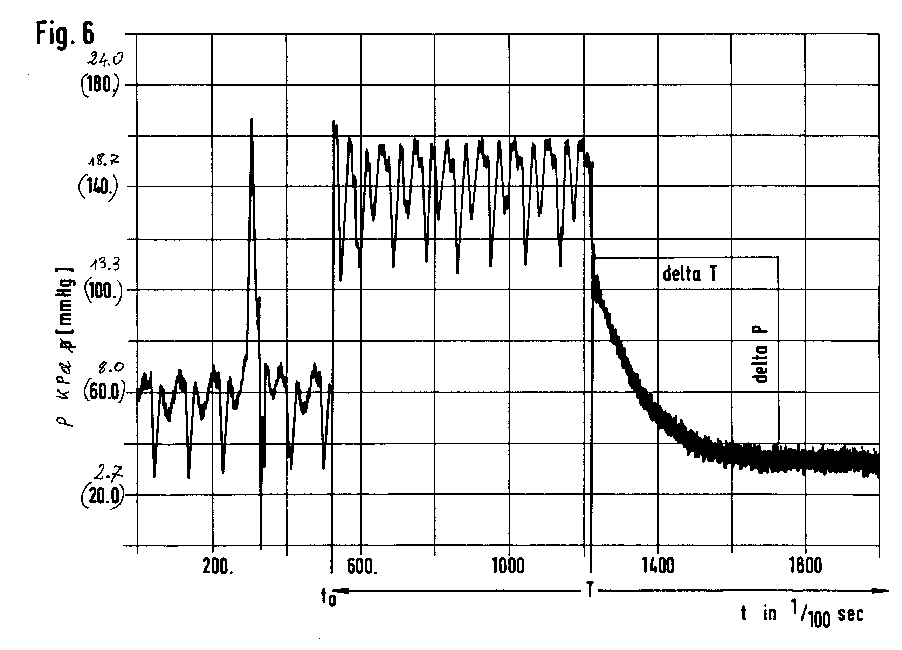

Das erfindungsgemäße Verfahren soll nun anhand der Kurvenverläufe gemäß den Figuren 5 und 6 erläutert werden. In diesen Figuren ist jeweils der zeitliche Verlauf des Dialysat-Druckes p dargestellt, bei einer Prüfzeit von T=15 sec.. Die Fig. 5 zeigt den Druckverlauf bei intaktem Kontrollsystem, die Fig. 6 den entsprechenden Verlauf bei einem Leck von 2,7 ml/min.

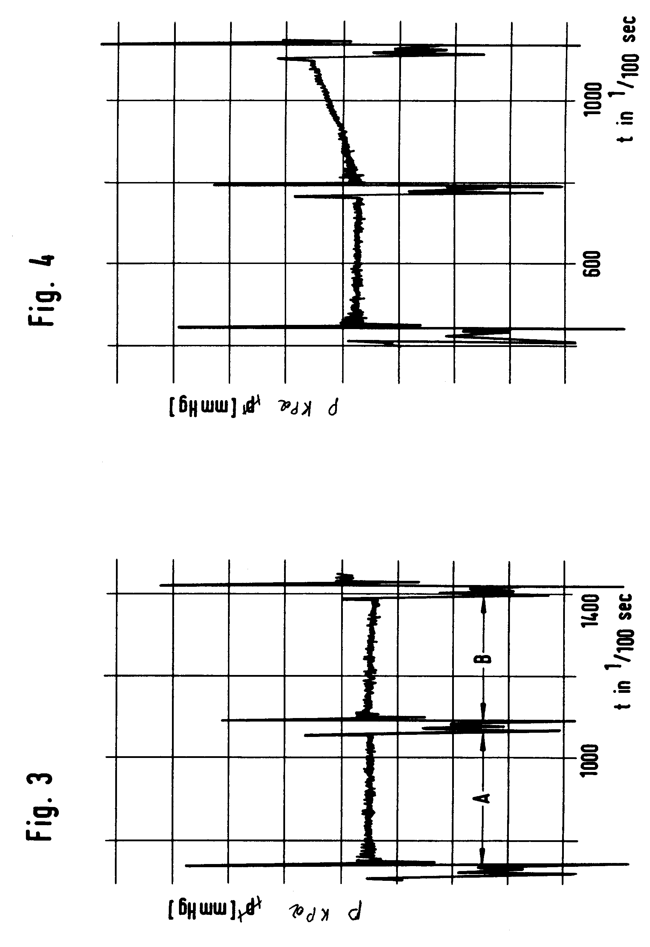

In dem linken Teil der Figuren ist jeweils der Druckverlauf aufgrund des Arbeitens der Bilanzkammern 14 dargestellt, und zwar für zwei Arbeitstakte A und B. Die Druckspitzen rühren vom Umschalten der Arbeitstakte her. Dieser Kurventeil wird später noch anhand der Fig. 3 und 4 beschrieben. Bei $t_o$ wird der Dialysator 1 für die Dauer des Prüfintervalles T vom Dialysierflüssigkeitskreislauf getrennt, indem die Dialysatorventile 17a, 17b geschlossen und das Bypassventil 17c geöffnet wird. Der Druck steigt jeweils zunächst an, gleicht sich aus und bleibt im Fall eines intakten Systems stabil (Fig. 5). Im Fall eines Lecks dagegen fällt er ab. Dieser Druckabfall, wie er deutlich in Fig. 6 zum Ausdruck kommt, ist ein eindeutiger Indikator dafür, daß das UF-Kontrollsystem nicht mehr integer ist.

Während des Druckhaltetestes ist der Dialysator zwar abgeschaltet, jedoch ist für eine vorgegebene Zeitdauer die Situation hinsichtlich des Betriebsdruckes hinreichend realistisch. Typischerweise sollte der Druckhaltetest innerhalb eines Zeitintervalles von max. 20 sec. durchgeführt werden.

Da ein Fehler zu einem beliebigen Zeitpunkt während der Behandlung auftreten kann, muß der Test periodisch durchgeführt werden. Diese Periode hängt vom UFK des Dialysators ab. Es ist daher in einer Ausgestaltung der Erfindung zweckmäßig, das Intervall zwischen zwei Druckhaltetests vom UFK des Dialysators abhängig zu machen. Denn bei einem hohen UFK wird die typischerweise für die Fehlultrafiltration gesetzte Grenze von 0,5 l bereits rasch erreicht, während bei einem niedrigen UFK die Periode gleich der Behandlungszeit sein kann, d.h. der Druckhaltetest überhaupt nicht durchgeführt werden muß, weil das Schutzsystem auf der Basis der Überwachung des Membrandruckes bereits anspricht.

Die Dauer der Periode kann wie folgt bestimmt werden: Ausgegangen sei von einem Grenzwert für den Ultrafiltrationsfehler von 0,5 l sowie der Auflösung des Transmembrandruckmonitors von 2,67 kPa (20 mmHg), d.h. der Monitor bei einer Abweichung von mehr als 2,67 kPa (20 mmHg) vom Sollwert das Hämodialyse-Gerät in den sicheren Zustand schaltet.

Die UF-Menge (ml) ist gleich dem Produkt aus Ultrafiltrationsrate UFR (ml/h) und Zeit t (min) :

$$UF = UFR * t/60 \qquad\qquad (1)$$

Die Ultrafiltrationsrate UFR ist gleich dem Produkt aus Ultrafiltrationskoeffizienten UFK und Druck p.

$$UFR = UFK * p \qquad\qquad (2)$$

Durch Einsetzen von (2) in (1) ergibt sich für die UF-Menge:

$$UF = UFK * p * t/60 \qquad\qquad (2a)$$

Für den maximalen Fehler ΔUF läßt sich die Periode Δt in Abhängigkeit vom UFK errechnen.
Es ergibt sich durch Auflösen der Gleichung (2a) nach Δt:

$$\Delta t = ( UF/p) * (60/UFK) \qquad\qquad (3)$$

Für einen UFK von 450L/h · kPa (60L/h · mmHg) und einen maximalen UF-Fehler von 500 ml und p von 2,67kPa (20 mmHg) durch Einsetzen dieser Werte in Gleichung (3) eine Periode -t von 25 min.

Aus Gleichung (3) läßt sich unter Auflösung dieser Gleichung nach UFK unschwer errechnen, ab welchem UFK keine Prüfung mehr erforderlich ist. Dafür ist in die Gleichung für -t die Dialyse-Behandlungszeit einzusetzen.

Daraus ergibt sich unter Einsetzen des Werts in die Gleichung:

$$UFK = (\Delta UF/p)*(60/\Delta t)$$

für eine Behandlungszeit von 4h ein UFK von ca. 45L/h • kPa (6L/h • mm Hg).

Beim Einsetzen von Dialysatoren mit einem UFK von größer als 45L/h • kPa (6L/h • mm Hg). wird daher das erfindungsgemäße Verfahren mit an den jeweiligen UFK angepaßten periodischen Zeitabständen durchgeführt.

Das erfindungsgemäße Verfahren kann manuell durchgeführt werden. In den von dem jeweiligen UFK des eingesetzten Dialysators vorgegebenen Zeitintervallen führt eine Bedienungsperson jeweils die Schaltvorgänge zum Bypassen des Dialysators aus und beobachtet visuell den Druckverlauf durch Beobachten der Anzeige des Drucksignales des Druckaufnehmers 22. Wird ein Leck erkannt, wird das Dialysegerät in den sicheren Zustand gefahren, ansonsten wird der Dialysator wieder angeschaltet. Bei dieser manuellen Prüfung ist eine Änderung des Dialysegerätes nicht erforderlich, d.h. das Verfahren kann dann auch bei vorhandenen Geräten durchgeführt werden.

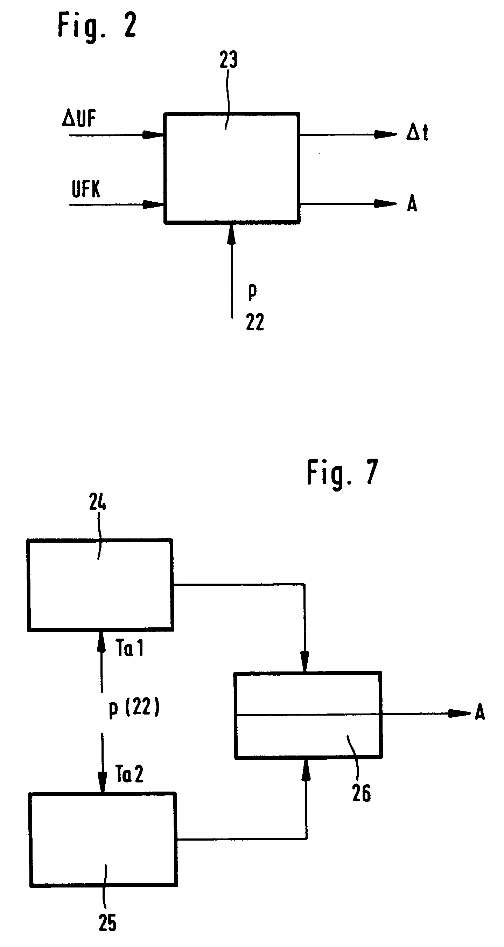

Die v.g. Schritte können grundsätzlich auch selbsttätig durchgeführt werden, z.B. unter Zuhilfenahme eines Mikroprozessorsytems 23 gemäß Fig. 2.

Dieses errechnet auf der Basis der Gleichung (3) unter Eingabe der gerätetypischen Werte -UF, UFK und dem Transmembrandruck die notwendige Prüfperiode -t und veranlaßt abhängig von dem Verlauf des (digitalisierten) Drucksignals des Sensors 22 die notwendigen Schaltmaßnahmen, Anzeigen und Alarmsignale über den Ausgang A.

Das beschriebene erfindungsgemäße Verfahren erlaubt es somit, periodisch alle Defekte im Ultrafiltrationssystem zu erkennen, die eine Fehlultrafiltration über der genannten Schwelle bewirken.

Nun hängt, wie beschrieben, der Abzug des Ultrafiltrates aus dem Dialysierflüssigkeitskreislauf II eng mit der Funktionsweise des Bilanzkammersystems 14 zusammen.

Man geht zwar in der Sicherheitstechnik für med. Geräte davon aus, daß im Behandlungsintervall nur ein einziger Fehler in einer Teileinrichtung (z.B. UF-Kontrolleinrichtung) auftritt, jedoch würde ein Verfahren zur Erkennung eines Defekts im Bilanzkammersystem 14 ohne Unterbrechung der Dialyse die Sicherheit erhöhen.

Das Bilanzkammersystem 14 ist, wie beschrieben, aus zwei gleichartigen Teilsystemen aufgebaut, die mit einer vorgegebenen Arbeitstaktperiode abwechselnd betrieben werden, um einen gleichmäßigen Fluß zu erzielen. An die Genauigkeit der Bilanziervorrichtung sind sehr hohe Anforderungen zu stellen. Tritt ein Defekt in einem der Systeme auf, so wird die Bilanzierung während des Arbeitstaktes dieses Teilsystems gestört. Dadurch kommt es zu einer kleinen Abweichung des Dialysierflüssigkeitsdruckes. Diese kleine Abweichung kann jedoch nicht detektiert werden, da der Druck durch Schwankungen, die von der Wirkung der Blutpumpe 4, der UF-Pumpe 21 aber auch der Umschaltung selbst herrühren. Um nun diese kleine Abweichung erkennbar zu machen, wird der Druck über jeweils einer Takt-Periode gemittelt und gespeichert. Die zu jeweils einem Bilanzkammersystem gesammelten Werte werden nun ihrerseits gemittelt und außerdem die Varianz bestimmt. Sobald die Varianz soweit gesunken ist, daß ein Vergleich mit erforderlicher Auflösung möglich ist, werden die gemittelten Werte der beiden Kammern verglichen und bei Abweichung größer einem bestimmten Betrag (typisch 0,53kPa (4 mmHg) bei UFK des Dialysators von 300L/h • kPa (40L/h • mmHg)) eine Alarmmeldung abgegeben. Dieser Ansprechwert kann nun vom UFK des Dialysators abhängig gemacht werden. Ein geringer UFK führt bei gleicher Leckrate bekanntlich zu einer größeren Druckdifferenz.

Fig. 3 zeigt den zeitlichen Verlauf des Dialysatdrucks p im mmHg bei intaktem Bilanziersystem über zwei Bilanzkammerintervalle A und B. Die Druckspitzen rühren von der Umschaltung der Bilanzkammern her. Wie man erkennt, verläuft der Druck in beiden Intervallen flach.

Fig. 4 zeigt einen Druckverlauf wie Fig. 3, jedoch ist ein Bilanzkammerventil mit einem Leck versehen, das zu einem Ultrafiltrationsfehler von ca. 2 ml/min führt. Wie man erkennt ist der Druckverlauf in einem Teil ansteigend, im anderen Teil flach. Daraus kann unschwer, wie oben beschrieben, auf ein Leck geschlossen werden.

Für die Schaltungstechnische Umsetzung der v.g. Abläufe stehen dem Fachmann eine Reihe von Mitteln, inbes. elektronischer und digitaler Art bzw. in Form eines Mikroprozessers zur Verfügung.

Die Fig. 7 zeigt in schematischer Blockbilddarstellung eine entsprechende Schaltung für eine der Bilanzkammern 14. Für die zweite der Bilanzkammern ist eine entsprechende Schaltung vorzusehen. Die Schaltung nach Fig. 7 weist zwei Stufen 24 und 25 auf, von denen die Stufe 24 dem Arbeitstakt 1, d.h. der einen Kammerhälfte der Bilanzkammer und die Stufe 25 dem Arbeitstakt 2, der anderen Kammerhälfte, zugeordnet ist. Diese Stufen speichern jeweils das geeignet aufbereitete und über die Taktperiode gemittelte Drucksignal p vom Druckaufnehmer 22. Entsprechende elektronische Schaltungen für diese Stufen zur Speicherung von Mittelwerten sind dem Fachmann bekannt.

Die zu jeweils einem Kammerteil gehörenden Werte werden in der nachgeschalteten Stufe 26 gespeichert und gemittelt, wobei die Mittelwerte in der Stufe miteinander verglichen werden. Sobald die Abweichung einen in der Stufe 26 vorgegebenen Wert überschreitet, wird am Ausgang A ein Alarmsignal erzeugt und ggf. ein Schaltvorgang ausgelöst. Falls zweckmäßig erfolgt gleichzeitig auch eine Anzeige. Auch für die Stufe 26 stehen dem Fachmann entsprechende Schaltungen zum Speichern, Mittelwertbilden und Vergleichen von Signalen, insbesondere solche auf digitaler Basis, zur Verfügung. Auch können sämtliche Stufen durch einen Mikroprozessor nachgebildet werden.

**Patentansprüche**

**1.** Verfahren zur Feststellung der Funktionsfähigkeit von Baugruppen im angrenzenden Dialysierflüssigkeitskreislauf (II) eines Dialysators (1) eines Hämodialysegerätes, mit einer volumetrischen Ultrafiltration im Dialysierflüssigkeitskreislauf (II) und einem Kontrollsystem (21a) für die Ultrafiltrationsrate in Verbindung mit einem Druckaufnehmer (22) im Dialysierflüssigkeitskreislauf (II) und einem Schutzsystem durch Überwachung des Transmembrandruckes, wobei der Druckverlauf im Dialysierflüssigkeitskreislauf (II) bei abgeschaltetem und überbrücktem Dialysator (1) durch Detektion der Signale des Druckaufnehmers (22) zur Feststellung der Funktionsfähigkeit herangezogen wird, dadurch gekennzeichnet, daß während der Dialyse in periodischen Zeitabständen der Dialysator (1) für jeweils ein kurzes Zeitintervall (T), in welchem der mittlere Dialysatbetriebsdruck im fehlerfreien Zustand stabil ist, vom Dialysierflüssigkeitskreislauf (II) abgetrennt wird und der Druckverlauf in der Dialysierflüssigkeit außerhalb des abgetrennten Dialysators auf Abweichung vom stabilen Zustand erfaßt wird.

2. Verfahren nach Anspruch 1 dadurch gekennzeichnet, daß das Zeitintervall, innerhalb dessen der Druckhaltetest durchgeführt wird, typischerweise im Bereich von 20 Sekunden liegt.

3. Verfahren nach Anspruch 1 oder 2, dadurch gekennzeichnet, daß die Periode zwischen zwei Druckhaltetest-Zeitintervallen abhängig von dem Ultrafiltrationskoeffezienten des eingesetzten Dialysators bestimmt wird nach der Gleichung

$$\Delta t = (\ \Delta UF/p \ast (60/UFK)$$

mit $\Delta t$ = Periode, $\Delta UF$ = max. Fehler der Ultrafiltration,
p = Transmembrandruck und UFK = Ultrafiltrationskoeffizient.

4. Verfahren nach einem der Ansprüche 1 bis 3, dadurch gekennzeichnet, daß es manuell unter visueller Beobachtung des Druckverlaufes durchgeführt wird.

5. Verfahren nach einem der Ansprüche 1 bis 3, dadurch gekennzeichnet, daß es selbsttätig durch das Hämodialysegerät durchgeführt wird.

6. Vorrichtung zur Durchführung des Verfahrens nach Anspruch 5, dadurch gekennzeichnet, daß im Hämodialysegerät ein Mikroprozessorsystem (23) vorgesehen ist, das während der Dialyse periodisch in vorgegebenen Zeitabständen Schaltsignale zur Trennung des Dialysators (1) vom Dialysierflüssigkeitskreislauf (II) für ein vorgegebenes Zeitintervall (T) erzeugt, dem neben dem Signal (p) des Druckaufnehmers (22) alle notwendigen Werte zugeführt sind und der abhängig vom Druckverlauf im Zeitintervall ein Anzeige-/Alarm-/Schaltsignal erzeugt.

7. Verfahren Zur Festellung der Funktionsfähigkeit von Baugruppen im angrenzenden Dialysierflüssigkeitskreislauf (II) eines Dialysators (1) eines Hämodialysegerätes, mit einem im Dialysierflüssigkeitskreislauf (II) angeordneten Bilanzkammersystem, bestehend aus zwei, jeweils geteilten Bilanzkammern (14), die jeweils gewährleisten, daß zum bzw. vom Dialysator (1) stets die gleiche Menge Dialysierflüssigkeit zu- bzw. abgeführt wird, und die jeweils in zwei aufeinanderfolgenden Arbeitstakten abwechselnd betrieben werden, und mit einem Druckaufnehmer im Dialysierflüssigkeitskreislauf (II), dadurch gekennzeichnet, daß für jeden Kammerteil die Druckverläufe am Druckaufnehmer (22) während jeweils einer Taktperiode gemittelt und in getrennten Speichern abgelegt werden, daß ferner die gespeicherten Druckwerte zugehöriger Kammerteile ihrerseits gemittelt und miteinander verglichen werden, wobei im Falle des Überschreitens einer bestimmten Differenz eine Anzeige / ein Schaltvorgang / eine Alarmauslösung erfolgt.

8. Verfahren nach Anspruch 7, dadurch gekennzeichnet, daß der Schwellwert abhängig vom Ultrafiltrationskoeffizienten des Dialysators (1) bestimmt wird.

9. Vorrichtung zur Durchführung des Verfahrens nach Anspruch 7 oder 8, dadurch gekennzeichnet, daß pro Bilanzkammer (14) eine elektronische Schaltung vorgesehen ist, die zwei Eingangsstufen (24, 25) aufweist, wobei jede Stufe einer Kammerhälfte fest zugeordnet ist und als Eingangssignal das Drucksignal des Druckaufnehmers (22) empfängt, und daß jede Eingangsstufe so ausgebildet ist, daß sie jeweils die Druckverläufe während der zugehörigen Arbeitstaktperiode mittelt und speichert und daß die elektronische Schaltung eine den Eingangsstufen (24, 25) nachgeschaltete Ausgangsstufe (26) aufweist, die so ausgebildet ist, daß sie für jedes Kammerteil die bei den einzelnen zugehörigen Taktperioden gemittelten Drucksignale über mehrere Taktperioden mittelt, die Mittelwerte miteinander vergleicht und ein Signal auslöst, wenn die Differenz einen vorgegebenen Schwellwert überschreitet.

10. Vorrichtung nach Anspruch 9, dadurch gekennzeichnet, daß die elektronische Schaltung durch ein Mikroprozessorsystem gebildet ist.

## Claims

1.  Procedure for the establishment of the functional capability of assemblies in the adjoining dialysing fluid circulation (II) of a dialyser (1) of a haemodialysis appliance, with a volumetric ultra-filtration in the dialysing fluid circulation (II) and a control system (21a) for the ultrafiltration in conjunction with a pressure recorder (22) in the dialysing fluid circulation (II) and a safety system through monitoring of the transmembrane pressure, in which the course of pressure in the dialysing fluid circulation (II) is adduced, with dialyser (1) switched off and bridged over, for establishment of the functional capability by detection of the signals of the pressure recorder (22), characterised by the fact that during the dialysis the dialyser (1) is separated from the dialysing fluid circulation (II) at periodic time intervals for a short space of time (T) in each case, in which the average dialysate operating pressure is stable in the fault-free state, and the course of pressure in the dialysing fluid outside the separated dialyser is registered for deviation from the stable state.

2.  Procedure as per claim 1, characterised by the fact that the space of time in which the pressure retention test is carried out is situated typically in the area of 20 seconds.

3.  Procedure as per claim 1 or 2, characterised by the fact that the period between two pressure retention test periods is determined depending on the ultrafiltration coefficient of the dialyser used according to the equation

$$\Delta t = ( UF/p * (60/UFK)$$

with $\Delta t$ = period, $\Delta UF$ = max. fault of the ultrafiltration,
p = transmembrane pressure and UFK = ultrafiltration coefficient.

4.  Procedure as per one of the claims 1 to 3, characterised by the fact that it is carried out manually with visual observation of the course of pressure.

5.  Procedure as per one of the claims 1 to 3, characterised by the fact that it is carried out automatically by the haemodialysis appliance.

6.  Device for the carrying out of the procedure as per claim 5, characterised by the fact that in the haemodialysis appliance a microprocessor system (23) is provided which produces periodically at preset time intervals switching signals for the separation of the dialyser (1) from the dialysing fluid circulation (II) for a preset space of time (T), to which besides the signal (p) of the pressure recorder (22) all necessary values are fed and which produces an indication/alarm switching signal depending on the course of pressure in the time interval.

7.  Procedure for the establishment of the functional capability of assemblies in the adjoining dialysing fluid circulation (II) of a dialyser (1) of a haemodialysis appliance, with a balance chamber system fitted in the dialysing fluid circulation (II) consisting of two separate balance chambers (14) which guarantee in each case that to or from the dialyser (1) the same quantity of dialysing fluid is fed in or run off and which are operated alternately in two successive work phases, and with a pressure recorder in the dialysing fluid circulation (II), characterised by the fact that for each part of the chamber the courses of pressure at the pressure recorder (22) are averaged during a phase period and are stored in separate memories, that further the stored pressure values of the appropriate parts of the chamber are averaged for their part and compared with each other, with an indication/a switching process/an alarm triggering being effected in the event of exceeding a specified difference.

8.  Procedure as per claim 7, characterised by the fact that the threshold value is determined depending on the ultrafiltration coefficient of the dialyser (1).

9.  Device for the carrying out of the procedure as per claim 7 or 8, characterised by the fact that for each balance chamber (14) an electronic circuit is provided which exhibits two input stages (23, 25) with each stage being fixedly allocated to one half of the chamber and receiving as input signal the pressure signal of the pressure recorder (22) and that each input stage is so formed that it in each case averages and stores the courses of pressure during the appropriate work phase period and that the electronic circuit exhibits an output stage (26) appended to the input stages (24, 25), which is so formed that for each part of the chamber it averages the pressure signals averaged in the individual appropriate phase periods over several phase periods, compares the average values with each other

9

and sets off a signal when the difference exceeds a preset threshold value.

**10.** Device as per claim 9, characterised by the fact that the electronic circuit is formed by a microprocessor.

## Revendications

**1.** Procédé de vérification du bon fonctionnement de sous-ensembles dans le circuit de liquide de dialyse (II) adjacent d'un dialyseur (1) d'un appareil d'hémodialyse, avec une ultrafiltration volumétrique dans le circuit de liquide de dialyse (II), un système de contrôle (21a) du débit d'ultrafiltration relié à un capteur de pression (22) placé dans le circuit de liquide de dialyse (II) et un système de protection par monitorage de la pression transmembranaire, l'évolution de la pression dans le circuit de liquide de dialyse (II), le dialyseur (1) étant mis hors service et court-circuité, par détection des signaux émis par le capteur de pression (22) étant utilisée pour déterminer la fonctionnalité, caractérisé en ce que le dialyseur (1) est isolé du circuit de liquide de dialyse (II) à intervalles périodiques pendant une courte durée (T) au cours de la dialyse et en ce que l'évolution de la pression du liquide de dialyse à l'extérieur du dialyseur isolé est enregistrée pour rechercher un éventuel écart par rapport à l'état stable.

**2.** Procédé selon la revendication 1, caractérisé en ce que l'intervalle de temps à l'intérieur duquel le test de maintien de la pression est effectué se situe typiquement dans la plage de 20 secondes.

**3.** Procédé selon la revendication 1 ou la revendication 2, caractérisé en ce que la période entre deux intervalles de temps pour le test de maintien de la pression est déterminée en fonction du coefficient d'ultrafiltration du dialyseur utilisé au moyen de l'équation suivante :

$$\Delta t = (\Delta UF/p * (60/CUF)$$

dans laquelle $\Delta t$ est la période, $\Delta UF$ est le nombre maximum de mauvaises ultrafiltrations, p est la pression transmembranaire, et CUF est le coefficient d'ultrafiltration.

**4.** Procédé selon l'une quelconque des revendications 1 à 3, caractérisé en ce que ledit procédé est mis en oeuvre manuellement par observation visuelle de l'évolution de la pression.

**5.** Procédé selon l'une quelconque des revendications 1 à 3, caractérisé en ce que ledit procédé est mis en oeuvre automatiquement par l'appareil d'hémodialyse.

**6.** Dispositif pour la mise en oeuvre du procédé selon la revendication 5, caractérisé en ce que l'appareil d'hémodialyse comporte un système de microprocesseur (23) qui génère périodiquement à des intervalles de temps prédéterminés au cours de la dialyse des signaux de commande destinés à isoler le dialyseur (1) du circuit de liquide de dialyse (II) pendant une durée prédéterminée (T), qui reçoit, outre le signal (p) du capteur de pression (22), toutes les valeurs nécessaires, et qui génère un signal d'affichage, d'alarme et/ou de commutation en fonction de l'évolution de la pression pendant ladite durée.

**7.** Procédé de vérification du bon fonctionnement de sous-ensembles dans le circuit de liquide de dialyse (II) adjacent d'un dialyseur (1) d'un appareil d'hémodialyse, avec un système de chambres d'équilibrage disposé dans le circuit de liquide de dialyse (II) et composé de deux chambres d'équilibrage (14) l'une et l'autre divisées qui garantissent qu'une quantité égale de liquide de dialyse est constamment amenée au dialyseur (1) ou évacuée de celui-ci et qui sont alternativement mises en jeu selon deux cycles de travail consécutifs, et avec un capteur de pression placé dans le circuit de liquide de dialyse (II), caractérisé en ce que l'évolution des pressions mesurée par le capteur de pression (22) dans chaque partie de chambre est moyennée et mémorisée dans des dispositifs de stockage séparés, et en ce que les valeurs de pression mémorisées pour les parties de chambre concernées sont également moyennées de leur côté et comparées les unes avec les autres, le dépassement d'une différence donnée provoquant un affichage, une opération de commutation ou le déclenchement d'une alarme.

**8.** Procédé selon la revendication 7, caractérisé en ce que la valeur seuil est déterminée en fonction du coefficient d'ultrafiltration du dialyseur (1).

**9.** Dispositif pour la mise en oeuvre du procédé selon la revendication 7 ou la revendication 8, caractérisé en ce qu'il est prévu pour chaque chambre d'équilibrage (14) un circuit électronique qui présente deux étages d'entrée (24, 25), chaque étage étant affecté de manière fixe à une moitié de chambre et recevant le signal du capteur de pression (22), en ce que chaque étage d'entrée est conçu de manière à moyenner les profils de pression au cours de la période concernée du cycle de travail et à les mémoriser, et en ce que le circuit électronique comporte un étage de sortie (26) disposé à la suite des étages d'entrée (24, 25) et conçu de manière à moyenner sur plusieurs périodes de cycle les signaux de pression moyennés sur les différentes périodes de cycle correspondantes, à comparer les valeurs moyennes entre elles et à générer un signal lorsque la différence est supérieure à une valeur seuil prédéterminée.

10. Dispositif selon la revendication 9, caractérisé en ce que le circuit électronique est formé par un microprocesseur.

Fig. 1

# Fig. 2

# Fig. 7

Fig. 3

Fig. 4

p kPa ↑p↑ [mmHg]

t in ¹/₁₀₀ sec

p kPa ↑p↑ [mmHg]

t in ¹/₁₀₀ sec

1000   1400

600   1000

A   B

EP 0 604 753 B1

Fig. 5

Fig. 6